# EUROPÄISCHE PATENTSCHRIFT

(11) **EP 2 028 960 B1**
(45) Veröffentlichungstag und Bekanntmachung des Hinweises auf die Patenterteilung: **24.11.2010**
(21) Anmeldenummer: 07725270.8
(22) Anmeldetag: 16.05.2007
(51) Int. Cl.: A44B 18/00, A61F 13/62

(54) **VERFAHREN ZUM ERREICHEN EINER DUFTABGABE BEI HAFTVERSCHLUSSTEILEN**
METHOD FOR ACHIEVING A FRAGRANCE RELEASE WITH SEALING PARTS
PROCÉDÉ PERMETTANT D'OBTENIR UNE DÉLIVRANCE DE PARFUM AVEC DES ARTICLES À FERMETURE ADHÉSIVE

(30) Priorität: 16.06.2006 DE 102006027849
(43) Veröffentlichungstag der Anmeldung: 04.03.2009
(73) Patentinhaber: Gottlieb Binder GmbH & Co. KG, 71088 Holzgerlingen (DE)
(72) Erfinder: TUMA, Jan, 71083 Herrenberg-Mönchberg (DE)
(74) Vertreter: Bartels, Martin Erich Arthur
(86) Internationale Anmeldenummer: PCT/EP2007/004354
(87) Internationale Veröffentlichungsnummer: WO 2007/144056

(56) Entgegenhaltungen:
- EP-A- 1 336 349
- WO-A-99/39675

## Beschreibung

Die Erfindung betrifft ein Verfahren zum Erreichen einer Duftabgabe bei Haftverschlußteilen, die mit korrespondierend ausgebildeten Haftverschlußteilen einen wiederholt öffen- und schließbaren Haftverschluß ausbilden, wobei das jeweilige Haftverschlußteil aus einem plastifizierbaren Kunststoffmaterial mittels einer Extrudereinrichtung hergestellt wird.

Durch die DE 697 24 657 T2 ist es bekannt, zwischen korrespondierenden Haftverschlußteilen einer-Baby- oder Inkontinenzwindel ein Bindemittel einzubringen, in das Duftkapseln aufgenommen sind. Werden die dahingehenden Haftverschlußteile voneinander getrennt, werden die Duftkapseln zumindest teilweise aufgerissen und geben an die Umgebung einen angenehm riechenden Duftstoff ab. Mit der dahingehenden Lösung ist es dem Verbraucher möglich, den schlechten Körpergeruch zu identifizieren, der das Erfordernis des Wechsels einer dahingehenden Windel anzeigt; beim Zusammenlegen der gebrauchten Windel unter Einsatz der jeweiligen Haftverschlußteile braucht dann aber der Verbraucher nicht permanent dem schlechten Geruch ausgesetzt zu sein, sondern dieser wird vielmehr nun kaschiert durch die Abgabe des Duftstoffes mittels der aufgerissenen Duftstoffkapseln. Eine Permanentbeduftung ist mit dem bekannten windel-Duftsystem nicht möglich.

Durch das DE 202 10 546 U1 ist es bekannt, Bekleidungstextilien mit auswechselbaren Pheromonduftstoffträgern zu versehen, in dem die dahingehenden Pheromone, vorzugsweise auf einem textilen Abgabeträger angebracht mittels sogenannter Kletten^{®}-Haftverschlüssen an den Bekleidungstextilien befestigt werden. Ist der Pheromonduftstoff verbraucht, also abgeduftet, kann mittels eines neuen Klettenhaftverschlußbandes mit dem separaten Pheromonduftstoffträger dieser erneut an der textilen Bekleidung angebracht werden. Durch den Einsatz der spezifisch auszuwählenden Pheromone soll der Träger des Bekleidungsstückes gegenüber seinen Mitmenschen erotisierend wirken. Das Gebrauchsmuster beschreibt dergestalt auch die Abgabe konventioneller Duft- und Aromastoffe über das genannte Trägersystem. Die dahingehend bekannte Lösung ist teuer in der Realisierung und der jeweils zum Einsatz kommende Trägerhaftverschluß verbraucht sich rasch.

Durch die WO-A-9939675 ist ein gattungsgemäßes Verfahren zum Erreichen einer Duftabgabe bekannt, wobei ein Duftstoff absorbierendes Material aus einem Compound besteht, bei dem ein Zeolithe-Werkstoff in eine Polymermatrix eingebunden ist. Die Polymermatrix verfügt über funktionelle Gruppen, die mit dem Zelolithe-Werkstoff reagieren und ihn dergestalt in die Matrix einbinden können. Das derart hergestellte Duftstoff absorbierende Material kann in dünne Filme oder Fasern extrudiert werden, was deren Anwendung besonders im Bereich von Tampons, Babywindeln und sanitären Tüchern als sinnvoll erscheinen läßt. Mit diesen bekannten Produkten läßt sich eine permanente Duftstoffabgabe erreichen, so dass insbesondere persönliche, unangenehme Geruchsausscheidungen überdeckt werden können.

Ausgehend von diesem Stand der Technik liegt daher der Erfindung die Aufgabe zugrunde, ein Verfahren zum Erreichen einer Duftabgabe bei Haftverschlußteilen zur Verfügung zu stellen, das eine permanente Duftstoffabgabe auch über längere Zeiträume sicherstellt, kostengünstig in der Realisierung ist, sowie funktionssicher im Gebrauch. Eine dahingehende Aufgabe löst ein Verfahren mit den Merkmalen des Patentanspruches 1 in seiner Gesamtheit.

Dadurch, dass gemäß dem kennzeichnenden Teil des Patentanspruches 1 das Kunststoffmaterial intrinsisch mit mindestens einem Duftstoff versehen wird, in dem der jeweilige Duftstoff der Extrudereinrichtung zum Herstellen des Haftverschlußteiles zugeführt wird, läßt sich der jeweilige Duftstoff fein dispergiert in hoher Konzentration in dem Kunststoffmaterial des Klettenhaftverschlußteiles unterbringen, so dass eine äußerst wirksame riechbare Duftstoffabgabe mit der erfindungsgemäßen Lösung möglich ist. Da sich der Duftstoff gleichmäßig im Kunststoffmaterial des Haftverschlußteiles verteilt, besteht darüber hinaus die Möglichkeit der Duftstoffabgabe über einen großen Oberflächenbereich des Haftverschlußteiles. Durch die intrinsische Einbettung des Duftstoffes im Kunststoffmaterial des Haftverschlußteiles ist darüber hinaus eine lang andauernde und mithin permanent wirkende Duftstoffabgabe möglich. Auch wenn das dahingehende Kletten^{®}-Haftverschlußteil in der Bekleidungsindustrie eingesetzt wird, beeinträchtigt ein Waschen der Textilien nicht die Duftstoffabgabe, so dass auch nach mehreren Waschvorgängen, das nach dem erfindungsgemäßen Verfahren erzeugte Haftverschtußteit die Duftstoffabgabe beim Tragen der Bekleidung ermöglicht.

Da für die Herstellung des Haftverschlußteiles ohnehin eine Extrudereinrichtung notwendig wird, läßt sich das erfindungsgemäße Verfahren in kostengünstiger Weise durchführen und es muss nicht noch erst wie im Stand der Technik aufgezeigt, für den jeweiligen Duftstoff eine komplizierte Mikroverkapselung vorgenommen werden, nach deren Auftrennen oder Zerreißen erst die Duftstoffabgabe möglich wird. Als Duftstoff im Sinne dieser Erfindung sollen alle riechenden Medien gelten wie Parfums, Aromen, reizende Duftmedien als Warnhinweis etc. aber auch nichtriechende Substanzen wie Hormone oder Botenstoffe (Pheromone).

Das erfindungsgemäße Verfahren lässt sich im sogenannten "Masterbatch" durchführen, d.h. der jeweilige Duftstoff wird gegebenenfalls zusammen mit einem Trägerpartikelmedium der Extrudereinrichtung eingangsseitig zugeführt zusammen mit dem granulatförmigen Kunststoffmaterial, wobei sich dann Duftstoff- und Kunststoffmaterial erst in der Plastifizierzone der Extrudereinrichtung intrinsisch aneinanderbinden. Ein besonders schonender Eintrag für den Duftstoff nebst etwaigen Trägerpartikeln besteht auch darin, in der sogenannten Vergleichmässigungszone der Extrudereinrichtung den jeweiligen Duftstoff einzubringen.

Bei einer besonders bevorzugten Ausführungsform des erfindungsgemäßen Verfahrens ist vorgesehen, das Haftverschlußteil in mehreren Schichten vorzugsweise laminar aufzubauen, unter Einsatz einer Koextrusionseinrichtung mit mehreren Abgabedüsen, wobei dann mindestens eine Schicht des Schichtenaufbaus den jeweiligen Duftstoff aufweisen kann oder bevorzugt ganz aus dem Duftstoffmaterial besteht. Im letztgenannten Fall ist dann vorzugsweise der Duftstoff an das Duftträgerpartikelmaterial gebunden.

Weitere vorteilhafte Ausführungsformeri der erfindungsgemäßen Verfahrenslösung sind Gegenstand der sonstigen Unteransprüche.

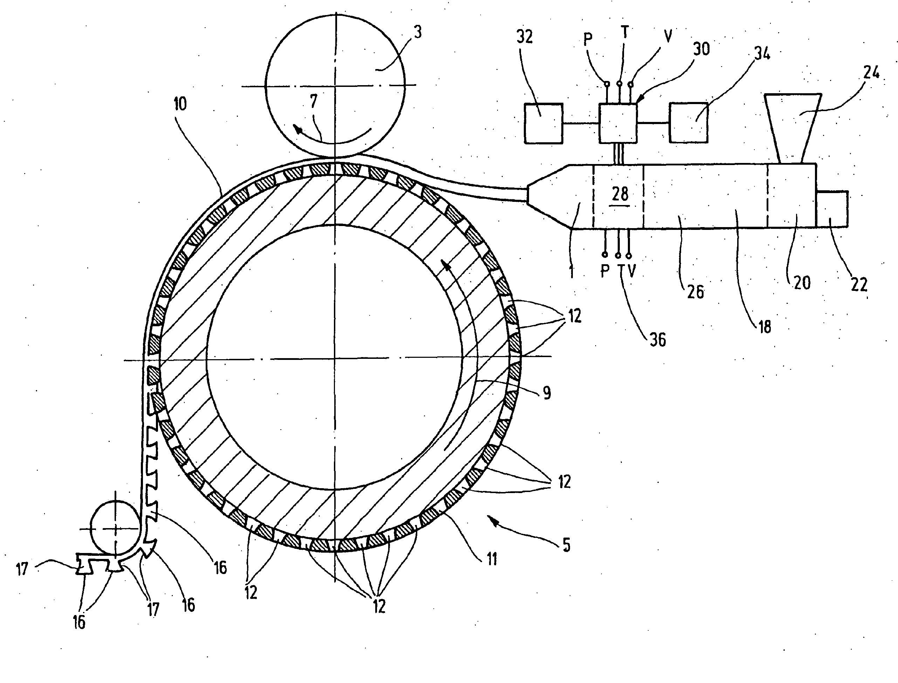

Im Folgenden wird das erfindungsgemäße Verfahren anhand einer Ausführungsform nach der einzigen Figur näher erläutert. Dabei zeigt in prinzipieller und nicht maßstäblicher Darstellung die dahingehende Figur eine stark schematisch vereinfacht und teils geschnitten gezeichnete Seitenansicht einer Vorrichtung, zur Durchführung des erfindungsgemäßen Verfahrens.

Die Figur zeigt in schematischer Darstellung Teile einer Vorrichtung zum Durchführen des erfindungsgemäßen Verfahrens mit einem Extruderkopf 1 als Zuführeinrichtung für in plastischem oder flüssigem Zustand befindlichen, insbesondere thermoplastischen Kunststoffmaterial, das als ein Band, dessen Breite derjenigen des herzustellenden Haftverschlußteils entspricht, einem Spalt zwischen einem Druckwerkzeug 3 und einem Formwerkzeug 5 zugeführt wird. Als Druckwerkzeug 3 ist eine Druckwalze vorgesehen und bei dem als Ganzes mit 5 bezeichneten Formwerkzeug handelt es sich um eine Formwalze. Beide Walzen sind in der Figur mit Bogenpfeilen 7 und 9 angegebenen Drehrichtungen angetrieben, so dass zwischen ihnen ein Förderspalt gebildet wird, durch den das Kunststoffband in Transportrichtung gefördert wird, während gleichzeitig Material zum Trägerband 10 des Haftverschlußteils geformt wird und das Trägerband 10 an der an der Formwalze anliegenden Seite durch die formgebenden Elemente der Formwalze, die zur Bildung von Verhakungsmitteln (Haftverschlußelemente in Form von Formteilen) erforderliche Formgebung erhält.

Zu diesem Zweck weist die Formwalze 5 am Umfang ein Sieb 11 auf mit einzelnen Formhohlräumen 12. Des weiteren sind die Formhohlräume 12, was nicht näher dargestellt ist, über die Formwalze mit ihrem Sieb 11 außenumfangseitig regelmäßig verteilt, wobei die Verteilung und die Anzahl frei wählbar sind. Insbesondere sind die Formhohlräume 12 mit Begrenzungswänden konvexen Bahnverlaufs versehen, so dass eine Art Hyperboloidstruktur entsteht, die dem herzustellenden Formelement entspricht. Mit den genannten Formhohlräumen 12 ist es also möglich Verhakungselemente oder Haftverschlußelemente herzustellen in Form jeweils eines, mit einem Kopfteil 16 versehenen Stielteils 17. Ein dahingehender Herstellaufbau für Verhakungsmittel oder sonstige Haftverschlußelemente ist üblich, und beispielsweise eingehend in der WO 02/13647 A2 beschrieben, so dass an dieser Stelle hierauf nicht mehr näher eingegangen wird.

Die hier für das jeweilige Haftverschlußteil zum Einsatz kommenden zu verarbeitenden Kunststoffmaterialien können vielfältiger Natur sein, beispielsweise in Form von Polyamiden oder Polyolefinen, wie Polypropylen oder Polyethylen (HDPL und LDPL). Ferner kommen auch andere Thermoplaste zum Einsatz, wie Polyester, Polyethylenterephthalat, Polystyrene, Polycarbonate, Polymethylmethacrylate, Ethylen, Vinylacetat Copolymere einschließlich Acrylat modifizierte Ethylene, Vinylacetat Polymere und Ethylen Acryl Azid Copolymere sowie Polyethylenstyrene. Ferner ist die Anwendung von Duroplasten denkbar, sowie von Elastomeren, wie natürlich oder synthetisch herstellbarer Gummi einschließlich von Styren Block Copolymeren mit Anteilen von Isopren, Butadien oder Ethylen (Butylene)-Blocks. Ferner besteht Verwendung für metalocen-catalisiertes Polyolefin, Polyurethan oder Polydiorganosiloxane. Zur Aussteifung der Trägerbahn 10 und zur Verstärkung können duktile Thermoplaste anwendbar sein, wie Nylon oder Polyvinylchlorid. Der jeweils hergestellte Formelementartikel in Form des Haftverschlußteils läßt sich auch mit Beschichtungen und Coatings versehen, die sich auch Aufdampfen und Aufrakeln lassen. Ferner sind zur Herstellung einer Strukturierung, im Sinne von selbstabreinigenden Oberflächen Nachbehandlungen möglich, sei es mit Laser, Ultraschall oder dergleichen mehr. Insbesondere können Herstellmaterialien eingesetzt werden, die biologisch abbaubar sind und insbesondere solche, die sich gut mit Duftstoffen intrinsisch versehen lassen.

Der bereits genannte Extruderkopf 1 mit Extruderdüse ist Teil einer als Ganzes mit 18 bezeichneten Extrudereinrichtung. Die dahingehende Extrudereinrichtung 18 weist eine nicht näher dargestellte Extruderschnecke auf, die sich von einer Eingangszone 20 bis zum Extruderkopf 1 mit Extruderdüse erstreckt. Die dahingehende Extruderschnecke wird über einen Antrieb, beispielsweise in Form eines Elektromotors 22 kontinuierlich angetrieben. An die Eingangszone 20 ist eine Eingangsstelle 24 für die Zufuhr des nicht näher dargestellten Kunststoffgranulats, beispielsweise in Form eines thermoplastischen Kunststoffmaterials angeschlossen. Innerhalb der Extrudereinrichtung 18 schließt sich an die Eingangszone 20 eine Aufwärm- und Verdichtungszone 26 an, die an ihrer der Eingangszone 20 abgewandten Seite in eine Vergleichmässigungszone 28 mündet, an die sich dann wiederum der Extruderkopf 1 mit Extruderdüse anschließt. Die Vergleichmässigungszone 28 hat insbesondere die Aufgabe, etwaig verdichtetes Kunststoffmaterial von der Homogenität her wieder zu vergleichmässigen, bevor das dahingehende Material ausgetragen wird. Der dahingehende Aufbau einer Extrudereinrichtung 18 ist üblich, so dass an dieser Stelle hierauf nicht mehr näher eingegangen wird. In dieser Konfiguration ist eine Standard-Extrudereinrichtung geschaffen, wobei es möglich ist, im sogenannten Masterbatch-Betrieb über die Eingangsstelle 24 den Duftstoff zusammen mit dem eingesetzten Kunststoffmaterial zuzuführen.

Es besteht aber auch gemäß der Darstellung nach der Figur die Möglichkeit, an die Vergleichmässigungszone 28 eine als Ganzes mit 30 bezeichnete Dosiereinrichtung für die Duftstoffabgabe anzuschließen, wobei die dahingehende Einrichtung in der Art einer Blackbox-Darstellung vereinfacht wiedergegeben ist. Neben einer Steuereinrichtung 32 weist die Dosiereinrichtung 30 noch mindestens einen Vorratsbehälter 34 für Duftstoffe auf. Des weiteren münden in die Vergleichmässigungszone 28 Sensoren für Druck p, Temperatur T und eventuell für die Geschwindigkeit v des eingesetzten Kunststoffmaterials mit ein, wobei die dahingehenden Ausgänge der Sensoren 36 an Eingänge der Dosiereinrichtung 30 sinnfällig angeschlossen sind. Neben den genannten Sensoren können noch weitere Sensoren eingesetzt werden (nicht dargestellt), beispielsweise für Viskosität, Linearität des Durchströmverhaltens etc.. Vorzugsweise ist ferner vorgesehen an verschiedenen Stellen Drucksensoren anzuordnen, um dergestalt Druckdifferenzen ermitteln zu können, für die sinnfällige Ansteuerung der Dosiervorrichtung 30 zum Erhalt eines gleichmässigen intrinsischen Duftstoffeintrages in das plastifizierte Kunststoffmaterial.

Mittels der Sensoren 36 wird jedenfalls der jeweilige Betriebszustand des Kunststoffmaterials in der Vergleichmässigungszone 28 erfasst und hiervon abhängig, speist die Steuereinrichtung 32 im Behälter 34 bevorratetes Duftstoffmittel in die Vergleichmässigungszone 28 der Extrudereinrichtung 18 ein. Kommt es eigenschaftsbedingt zu Änderungen an dem Kunststoffmaterial beispielsweise zu einer Verdichtung desselben, wird dann über die Dosiereinrichtung 30 weniger Duftstoff zugegeben als wenn das Kunststoffmaterial nicht zu dicht gepackt ist.

Auf diese Art und Weise wird über die Dosiereinrichtung 30 sichergestellt, das immer gleichförmig Duftstoffe in das Kunststoffmaterial gelangt, was später beim fertigen Endprodukt dazu führt, dass der Duftstoff durchgehend homogen eingetragen ist. Ferner erlaubt die Dosiereinrichtung 30 gegebenenfalls aus mehreren, nicht näher dargestellten Vorratsbehältern 34 verschiedene Arten an Duftstoffen zu entnehmen, diese zu mischen und dann in gemischter Form gleichzeitig oder alternierend in das Kunststoffmaterial einzubringen. Sofern die Trägerbahn 10 oder das Haftverschlußteil als Ganzes im Koextrusionsverfahren mehrschichtig aufgebaut werden soll, kann jeder Schicht eine eigene Extrudereinrichtung (nicht dargestellt) zugeordnet sein mit eigener Dosiereinrichtung für die Duftstoffzugabe, so dass sich jeder Schicht im Grunde nach eine eigene Duftstoffgebung zuordnen lassen würde. Da die Wegstrecke zwischen Vergleichmässigungszone 28 und Extruderkopf 1 sehr kurz ist, würde bei einem etwaigen Duftstoffwechsel ein unmittelbares Umsteigen auf den nächsten Duftstoff direkt möglich sein, wobei sich dergestalt die Ausschußrate an nicht gewünschter Duftstoffgebung oder beduftetem Material sich weitgehend reduzieren lässt.

Als Parfümöle bzw. Duftstoffe können im Rahmen der vorliegenden Erfindung einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8-18 C-Atomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methyl-cedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene, wie Limonen und Pinen.

Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller, Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl.

Ferner eignen sich für den Einsatz bei Haftverschlußteilen sogenannte haftfeste Riechstoffe, beispielsweise in Form von ätherischen Ölen, wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagöl, Kardamomenöl, Kassiaöl, Kiefernnadelöl, Kopaivabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Orangenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronenöl sowie Zypressenöl.

Ferner können auch sogenannte höhersiedende oder feste Riechstoffe natürlichen oder synthetischen Ursprungs als Duftstoff eingesetzt werden. Zu diesen Verbindungen zählen die nachfolgend genannten Verbindungen sowie Mischungen aus diesen: Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, α-Bromstyrol, n-Decylaldehyd, n-Dodecylaidehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jamson, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-βnaphtylketon, Methyl-n-nonylacetaldehyd, Methyl-n-nonylketon, Muskon, β-Naphtholethylether, β-Naphtholmethylether, Nerol Nitrobenzol, n-Nonylaldehyd, Nonylalkohol, n-Octylaldehyd, p-Oxy-Acethophenon, Pentadekanolid, β-Phenylethylalkohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undelacton, Vanilin, Veratrumaldehyd, Zimtaldehyd, Zimtalkohol, Simtsäure, Zimtsäureethylester, Zimtsäurebenzylester. Zu den leichter flüchtigen Riechstoffen zählen insbesondere die niedriger siedenden Riechstoffe natürlichen oder synthetischen Ursprung, die allein oder in Mischungen eingesetzt werden können. Beispiele für leichter flüchtige Riechstoffe sind Alkyisothiocyanate (Alkylsenföle), Butandion, Limonen, Linalool, Linaylacetat und -Propionat, Menthol, Menthon, Methyl-n-heptenon, Phellandren, Phenylacetaldehyd, Terpinylacetat, Zitral, Zitronellal.

Als besonders günstig hat sich zum Herstellen eines Zitronengeruches 3,7-Dimethyl-2,6-octadien-1-al erwiesen. Bei dem dahingehenden Geraniumaldehyd, das sich chemisch charakterisieren lässt als zweifach ungesättigter Monoterpenaldehyd, entsteht ein sehr ausgeprägter Zitronenduft. Der dahingehende Duftstoff ist unter der Angabe "Citral" im Markt bekannt und ist Hauptbestandteil der durch Wasserdampfdestillation erzeugten ätherischen Öle von Cymbopogon citratus STAPF (Lemongrasöl) und Litsea cubeba PERS. (May-Chang-Öl). Neben der Herstellung von Citral aus den beiden gut zugänglichen ätherischen Ölen, kann der Aldehyd wie dargelegt auch synthetisch durch Oxidation des korrespondierenden Alkohols Geraniol erzeugt werden.

Mit dem erfindungsgemäßen Verfahren lassen sich die genannten Duftstoffe unmittelbar verarbeiten; vorzugsweise besteht aber auch die Möglichkeit, die dahingehenden Duftstoffe an sogenannten Duftträgerpartikel anzugliedern. Die Raumform dahingehender Partikel kann beliebig sein und ist allein durch die technischen Möglichkeiten bei deren Herstellung und Verarbeitung beschränkt. Neben würfelförmigen und quaderförmigen Aufbauten sind mögliche Raumformen Kugeln, Zylinder aber auch reguläre Polyeder-Strukturen, beispielsweise in Form von Oktaedern oder Dodekaedern. Für den Erhalt einer großen wirksamen Oberfläche bietet sich auch die Aufrauhung der Partikeloberfläche an und/oder eine sternförmige Strahlenanordnung. Grundsätzlich kommt jedoch als Trägermaterial in Form der Duftträgerpartikel alle Stoffe in Frage, die in der Lage sind, die vorstehend genannten Duftstoffe zu adsorbieren und/oder zu absorbieren. Insbesondere als günstig haben sich organische Polymere erwiesen, aber z.B. auch poröse anorganische Materialien wie Kieselgele oder Zeolithe. Kieselgele oder Zeolithe werden beispielsweise in der Filtrationstechnik zur Erhöhung der Oberflächenwirkung von Filtersubstanzen bereits umfänglichst eingesetzt. Entsprechende Produkte könnten auch hier für den Duftstofftransport eingesetzt werden. Die genannten Trägerpartikel stabilisieren bei dem Extrudervorgang den Duftstoffeintrag.

Die eingesetzten Trägerpartikel können auch dazu dienen, weitere Eigenschaften an das Kunststoffmaterial des Haftverschlußteiles abzugeben. So könnten die Duftträgerpartikel beispielsweise zur Bekämpfung von Mikroorganismen wie Bakterien eingesetzt werden und dahingehend antimikrobielle Wirkstoffe enthalten. Solche Stoffe könnten beispielsweise übliche Bakterizide oder Fungizide sein. Besonders bevorzugt ist jedoch den jeweiligen Duftträgerpartikel entsprechend modifiziert für den Farbstofftransport in das Kunststoffmaterial des Haftverschlußteile einzusetzen. Insbesondere durch Zugabe von Flüssigfarbe über den Duftträgerpartikel an das Haftverschlußteil lässt sich dieses korrespondierend zu dem eingesetzten Duftstoff einfärben. Beispielsweise ist es für den Verbraucher günstig, wenn Partikel mit Zitronenduft eine gelbe Farbe aufweisen, während für Partikel mit Apfel- oder Kräuterduft häufig eine grüne Farbe bevorzugt wird. Bei der genannten Flüssigfarbe werden Farbpigmente verrieben, insbesondere pulverisiert in ein stabilisierendes Bindemittel platziert, wie Fettsäureester oder Fettsäureesterethoxylathe, Paraffinöle, Pflanzenöle, Weichmacher, mehrwertige Alkohole oder Amine sowie Alkoholethoxylathe. Es hat sich gezeigt, dass die dahingehenden Bindemittel auch eine gute Verbindung zu den genannten Duftträgerpartikeln eingehen.

Es hat sich für das durchzuführende Herstellverfahren als günstig erwiesen, die genannten Duftträgerpartikel im Bereich von 0,2 mm Durchmesser auszubilden und eine Füllung des Kunststoffmaterials mit Duftstoffen zwischen 5 bis 30% durchzuführen. Das erfindungsgemäße Verfahren lässt sich ausgesprochen kostengünstig durchführen und führt zu sehr großen Mengen an beduftetem Haftverschlußteilmaterial. Der Duftstoff lässt sich nicht abreiben und aufgrund seines intrinsischen Eintrages in das Haftverschlußteil ist mit einer lang anwährenden Beduftung der Umgebung zu rechnen. Insbesondere bei Verwendung von Zitrusdüften ist der verstärkte Einsatz an dahingehenden Kletten^{®}-Haftverschlüssen im Sanitärbereich möglich.

## Patentansprüche

1. Verfahren zum Erreichen einer Duftabgabe bei Haftverschlußteilen, die mit korrespondierend ausgebildeten Haftverschlußteilen einen wiederholt öffen- und schließbaren Haftverschluß ausbilden, wobei das jeweilige Haftverschlußteil aus einem plastifizierbaren Kunststoffmaterial mittels einer Extrudereinrichtung (18) hergestellt wird, **dadurch gekennzeichnet, dass** das Kunststoffmaterial intrinsisch mit mindestens einem Duftstoff versehen wird, in dem der jeweilige Duftstoff der Extrudereinrichtung (18) zugeführt wird.

2. Verfahren nach Anspruch 1, **dadurch gekennzeichnet, dass** der jeweilige Duftstoff an Duftträgerpartikel gekoppelt wird, die der Extrudereinrichtung (18) zugeführt werden.

3. Verfahren nach Anspruch 1 oder 2, **dadurch gekennzeichnet, dass** die Duftstoffzufuhr für die Extrudereinrichtung (18) als "Masterbatch"-Verfahren und/oder in der Vergleichmässigungszone (28) der Extrudereinrichtung (18) durchgeführt wird.

4. Verfahren nach einem der Ansprüche 1 bis 3, **dadurch gekennzeichnet, dass** mittels einer Dosiereinrichtung (30) abhängig vom Plastifizierungsgräd des Kunststoffmateriales und/oder der Temperatur (T) desselben, derart eine variable Menge an Duftstoff zugeführt wird, dass der Beduftungsgrad für das Haftverschlußteil gleichbleibend gestaltet wird.

5. Verfahren nach einem der Ansprüche 1 bis 4, **dadurch gekennzeichnet, dass** das Haftverschlußteil in Schichten aufgebaut mittels einer Koextrusionseinrichtung erhalten wird und dass mindestens eine Schicht den jeweiligen Duftstoff aufweist.

6. Verfahren nach einem der Ansprüche 1 bis 5, **dadurch gekennzeichnet, dass** die Duftträgerpartikel kleiner als 1 mm im Durchmesser sind, vorzugsweise 0,2 mm und in Kugelform eingesetzt werden.

7. Verfahren nach einem der Ansprüche 1 bis 6, **dadurch gekennzeichnet, dass** der jeweilige Duftstoff mit einem Farbstoff, insbesondere in Form von Flüssigfarbe verbunden wird.

8. Verfahren nach einem der Ansprüche 1 bis 7, **dadurch gekennzeichnet, dass** zwischen 5 und 30% der Einsatzmenge an Kunststoffmaterial Duftstoffe und Duftträgerpartikel eingesetzt werden.

## Claims

1. A method for achieving a fragrance release with sealing parts which with correspondingly formed sealing parts form a repeatedly openable and closeable seal, the respective sealing part being made of a plasticisable synthetic material by means of an extruder device (18), **characterised in that** the plastic material is provided intrinsically with at least one fragrance in which the respective fragrance is supplied to the extruder device (18).

2. The method according to Claim 1, **characterised in that** the respective fragrance is coupled to fragrance carrier particles which are supplied to the extruder device (18).

3. The method according to Claim 1 or 2, **characterised in that** the supply of fragrance for the extruder device (18) is implemented by the "master batch" method and/or in the equalisation zone (28) of the extruder device (18).

4. The method according to any of Claims 1 to 3, **characterised in that** by means of a metering device (30), dependently upon the plasticisation level of the plastic material and/or the temperature (T) of the latter, a variable quantity of fragrance is supplied such that the fragrance level of the sealing part remains consistent.

5. The method according to any of Claims 1 to 4, **characterised in that** the sealing part structured in layers is obtained by means of a coextrusion device, and that at least one level has the respective fragrance.

6. The method according to any of Claims 1 to 5, **characterised in that** the fragrance carrier particles have a diameter smaller than 1 mm, preferably 0.2 mm, and are used in spherical form.

7. The method according to any of Claims 1 to 6, **characterised in that** the respective fragrance is associated with a dye, in particular in the form of liquid dye.

8. The method according to any of Claims 1 to 7, **characterised in that** between 5 and 30% of the inputted quantity of plastic material, fragrances and fragrance carrier particles are used.

## Revendications

1. Procédé pour obtenir un dégagement de parfum dans des parties de fermeture autoagrippante, qui forment, avec des parties de fermeture autoagrippante constituées de manière correspondante, une fermeture autoagrippante pouvant s'ouvrir et se fermer de manière répétée, la partie respective de fermeture autoagrippante étant fabriquée en une matière plastique plastifiable au moyen d'un dispositif (18) d'extrusion, **caractérisé en ce que** l'on munit la matière plastique intrinsèquement d'au moins un parfum en envoyant le parfum respectif au dispositif (18) d'extrusion.

2. Procédé suivant la revendication 1, **caractérisé en ce que** l'on couple le parfum respectif à des particules de support de parfum, que l'on envoie au dispositif (18) d'extrusion.

3. Procédé suivant la revendication 1 ou 2, **caractérisé en ce que** l'on effectue l'envoi du parfum pour le dispositif (18) d'extrusion sous la forme d'un procédé "masterbatch" et/ou dans la zone (28) d'homogénéisation du dispositif (18) d'extrusion.

4. Procédé suivant l'une des revendications 1 à 3, **caractérisé en ce qu'**au moyen d'un dispositif (30) d'addition dosée, en fonction du degré de plastification de la matière plastique et/ou de sa température (T), on envoie une quantité variable de parfum, de manière à laisser constant le degré de parfumage de la partie de fermeture autoagrippante.

5. Procédé suivant l'une des revendications 1 à 4, **caractérisé en ce que** l'on obtient la partie de fermeture autoagrippante constituée en couche au moyen d'un dispositif de coextrusion et **en ce qu'**au moins une couche a le parfum respectif.

6. Procédé suivant l'une des revendications 1 à 5, **caractérisé en ce que** les parties de support de parfum ont un diamètre inférieur à 1 mm, de préférence inférieur à 0,2 mm, et sont utilisées sous forme de sphères.

7. Procédé suivant l'une des revendications 1 à 6, **caractérisé en ce que** l'on relie le parfum respectif à un colorant, notamment sous la forme de colorant liquide.

8. Procédé suivant l'une des revendications 1 à 7, **caractérisé en ce que** l'on utilise des parfums et des particules de support de parfum à raison de 5 à 30 % de la quantité de charge de matière plastique.
